# EUROPEAN PATENT SPECIFICATION

(11) **EP 3 388 188 B1**
(45) Date of publication and mention of the grant of the patent: **09.09.2020**
(21) Application number: 18164567.2
(22) Date of filing: 28.03.2018
(51) Int. Cl.: B23K 26/386, B23K 26/402, B23K 26/035, B23K 26/08, B23K 26/06, A61F 13/512

(54) **REVERSE DRAFT HOLE APPARATUS AND METHOD**
VORRICHTUNG UND VERFAHREN ZUM RÜCKWÄRTSZIEHEN VON LÖCHERN
APPAREIL ET PROCÉDÉ DE PERCAGE DE TROUS D'ASPIRATION INVERSÉS

(30) Priority: 29.03.2017 US 201762478420 P
(43) Date of publication of application: 17.10.2018
(73) Proprietor: Preco, Inc., Somerset, WI 54025 (US)
(72) Inventor: MILLER, Daniel, Roberts, WI Wisconsin 54023 (US); ARVEY, Ken, Emerald, WI Wisconsin 54013 (US); SLOAN, Donald R., Minnesota, MN Minnesota 55082 (US); DANDO, Austin, Wisconsin, WI Wisconsin 54023 (US)
(74) Representative: Downing, Michael Philip

(56) References cited:
- EP-A1- 0 549 357
- WO-A1-96/19313
- DE-A1-102013 226 792

## Description

### BACKGROUND

The present invention relates to laser processing of materials to form reservoirs in the material, and more particularly to the forming of reverse draft type holes for fluid in said materials.

Lasers are currently being used to modify materials in a vast array of products and markets. There are many lasers types and wavelengths available to choose from to best match performance desired in a given material and application. Lasers are currently used to form fluid wells or reservoirs in polyurethane foam. The foam is used across various fields, including the medical field where the foam is used in dressings for wound care to absorb drainage.

In the prior art, the foam material is perforated forming cylindrical wells or reservoirs, which are limited in the capacity to hold fluid relative to thickness of the material. Typical laser/galvanometer perforation creates a funnel or upside-down pyramid type shape, which limits the capacity of each reservoir, requiring more frequent dressing changes. WO 96/19313 discloses a method for producing holes or slots in layers of material intended for absorbent articles that requires irradiation with at least one focused electromagnetic beam. EP0549357 discloses an apparatus and method for fabricating a perforated web by light that requires an endless masking plate arranged above a continuously conveyed web.

### SUMMARY

An aspect of the present disclosure relates to an assembly for a laser processing system. The assembly is configured for forming a plurality of reverse draft holes, or reservoirs, in a material. The assembly comprises a housing configured to receive a galvanometer ("galvo") therein, and the housing has an exterior surface topography comprising a plurality of apertures. The assembly further comprises a compression mechanism comprising an exterior surface topography having a plurality of surface protrusions thereon for compressing the material passing over at least one of the plurality of surface protrusions. The housing and the compression mechanism are spaced apart providing a gap there between for receiving a web of the material therein.

The assembly may further be an assembly where the housing that is a first rotatable drum and the compression mechanism is a second rotatable drum.

The assembly may further be an assembly wherein the second drum comprises an under-material air delivery mechanism therein and configured to pressurize the foam material passing through the gap via an aperture in the protrusion on the drum.

The assembly may further be an assembly wherein the exterior surface topography of the compression mechanism is configured to compress the material passing through the gap and around the one or more apertures in the housing to compress the material within a laser processing area of the material. through the gap and around the one or more apertures in the housing to compress the material within a laser processing area of the material.

The assembly may further be an assembly wherein the laser processing area is a target area aligned with an axis of a laser beam directed by the galvo through at least one aperture in the housing to a surface of the material compressed adjacent thereto.

The assembly may further be an assembly wherein an under-material air delivery mechanism is positioned within the second drum such that at least one of the plurality of apertures in the first drum, a protrusion and respective aperture therein on the second drum, and an air flow path from the air delivery mechanism are all aligned with respect to the axis of the laser beam.

The assembly may further be an assembly wherein the housing comprises a perforation pattern comprising at least one row of apertures.

The assembly may further be an assembly wherein at least one of the surface protrusions of the compression mechanism is configured to align with and at least partially surround a corresponding aperture perimeter to provide a compressive force to the material passing through the gap near the corresponding aperture.

Another aspect of the present disclosure relates to a method of producing reservoirs in an open cell foam material with a laser processing system. The method comprises compressing the material around a target area on a first surface of the material and concurrently directing a focal point of a laser beam to the target area to produce the reservoirs having a bulging cross section.

The method may further comprise passing the material through a gap between a first housing and a compression mechanism, wherein the housing is configured to receive a laser steering mechanism therein and wherein the compression mechanism is configured to provide a compressive force to the material around the target area passing through the gap and aligning an aperture in an external surface of the housing with an external protrusion on the compression mechanism and further aligning the target area passing through the gap therewith; and directing the focal point of the laser beam from the housing and through the aperture and to the target area compressed by the compression mechanism for producing the reservoir.

The method may comprise the housing being a rotatable housing having a plurality of protrusions spaced apart on the external surface thereof and the compression mechanism being a movable and comprises a plurality of external protrusions and concurrently rotating the housing and moving the compression mechanism for continuously aligning an aperture in the external surface of the housing with a protrusion on the compression mechanism for producing a plurality of reservoirs on a moving web of material.

The method may comprise aligning an axis of the laser beam with the aperture and target area.

The method may optionally comprise providing a source of forced air below a travel path of the material through the gap for pressurizing the material at or near the target area for preventing a plume generated from laser processing from settling on the material.

The method may comprise laser processing the material, compressing the material and pressurizing the material at the target area and approximately concurrently.

### BRIEF DESCRIPTION OF THE DRAWINGS

FIG. 1 is side view of a reservoir formed by prior art laser processing apparatuses and methods.
FIG. 2 is a side view of a reverse draft hole formed by a laser processing apparatus of the present disclosure.
FIG. 3 is a side view of a laser processing system for forming the reverse draft holes of the present disclosure.
FIG. 4 is a side view of an air purge apparatus of the present disclosure.

### DETAILED DESCRIPTION

This disclosure relates to an assembly for a laser processing system for forming reverse draft holes in a material, and a method for producing a plurality of reverse draft holes in a material. Laser processing, for example, includes scoring and/or perforating the material or the creation of fluid wells or reservoirs in the material. It has been found that the dimensions of the fluid wells or reservoirs can be increased by compressing the foam in a unique way when laser processing the foam in order to create a reverse draft type of perforation shape in the foam.

Further, the discoloration and/or odor arising from the use of a laser system is reduced significantly or essentially eliminated by the optional use of an under-material air purge apparatus configured to pressurize individual cells of the foam material during laser processing. In one embodiment, the foam is compressed at a laser processing site concurrently with laser processing. Additionally, the foam may also be subject to positive pressure from an underside of the foam during processing.

This disclosure also includes articles made from foam material, particularly porous open cell materials processed by a laser and that is perforated with a plurality of reverse draft type holes. The articles may also be substantially discoloration-free as well as odor-free. The articles may include wound care bandages or other materials used for absorbing fluids.

A laser system or laser-based system as referred to herein relates to a system that processes material through the use of laser technology. Lasers provide a very efficient method of cutting, scoring, perforating or otherwise preparing material for various end uses over the old mechanical systems which may include die systems. Lasers cut, perforate and score through the use of a collimated amplified beam of light that terminates in a focal point. It is at the focal point of the beam that processing (e.g. cutting, perforating, scoring) typically takes place. Intense heat at the focal point processes the material in what can be described as essentially a burning process. A by-product of burning a polyurethane based foam material is the resulting discoloration, which may increase over time.

Although polyurethane-based foam materials are specifically mentioned herein, this disclosure encompasses other materials that can be processed by a laser for forming a plurality of reverse draft type holes or perforations in the material. The materials may be any type of open cell foam material or similar materials.

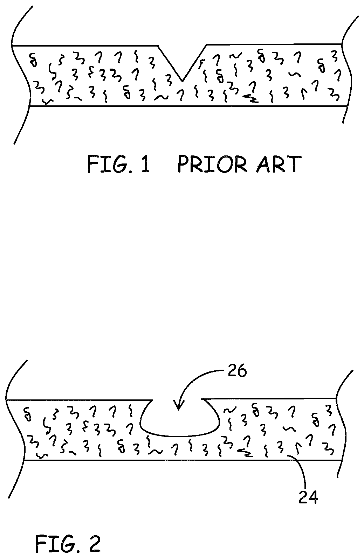

Prior art systems of perforating a foam material generally produce a cone shaped reservoir as illustrated in FIG. 1. The method and apparatus disclosed herein perforates the foam material and produces a reservoir having an increased capacity and shape referred to as a "reverse draft hole" illustrated at 26 in FIG. 2.

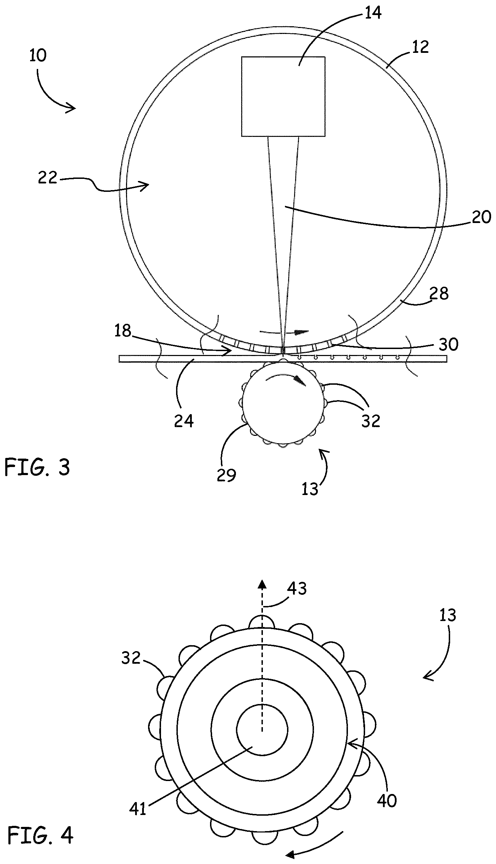

An assembly for a laser processing system for forming a plurality of reverse draft type hole 26 is illustrated generally at 10 in FIG. 3. The assembly 10 is part of laser processing system 100. The assembly 10 includes a housing 12 and at least one laser beam delivery system 14 is positioned inside the housing 12. The laser beam delivery system is a high-speed mechanism for delivering the laser beam to the desired target locations across the web. The laser beam delivery system may be a galvanometer ("galvo") or may otherwise comprise multiple lasers and/or beam splitters with individual lenses, stationary or moveable across the web in such a manner as to align with selected apertures. In an embodiment illustrated, the housing 12 is a rotatable drum 12, however other structures may be incorporated. The drum 12 is rotatably secured above a travel path 16 of the material 24 for laser processing.

A compression mechanism 13 is provided to the assembly 10 and may comprise a roller or a drum that is rotatably secured below the travel path 16 of the material 24 for laser processing. The drum 12 may be positioned directly above the roller or drum 13. The material 24 may be a polyurethane foam for laser processing or other open cell or similar foam-based material for perforating. The material 24 for laser processing (e.g., a web) passes underneath the upper drum 12 and galvo 14, and above the lower roller or drum 13 when being laser processed.

The assembly 10 is configured to concurrently provide compressive forces to the material while concurrently laser processing the material to produce reverse draft holes 26 in the material. The compressive force is applied to the material 24 as it passes through a laser processing window. The laser processing window is defined by the sweep of the galvo, or the area in which the galvo can direct the laser beam. Each of the upper drum 12 and lower roller or drum 13 may be rotatable to pass the material through the laser processing window and to compress the material passing there through. The upper drum 12 is positioned above the laser processing window and the lower roller or drum 13 is positioned below the laser processing window. Referring to throughout this disclosure also as a pair of drums 12 and 13, the drums may be positioned directly above and below the processing window, respectively.

The compressive force on the material is in part generated by the size of a gap (e.g. vertical distance) between the external surface of the upper drum 12 and lower roller or drum 13 as this gap may be smaller than the thickness of the material passing through the gap. The compressive force is more specifically provided to a laser processing site or target area 18 (e.g., the area of the material being laser processed) during laser processing. Providing compressive forces to the area near the processing site while laser processing the material to perforate the material produces a reservoir or well having an increased capacity. The reservoir shape 26 (exaggerated for illustration purposes) illustrated in the figures provides an increased fluid capacity to materials such as bandages, dressing and other absorbent products. More specifically, the reservoir shape is larger in volume than a cylindrical or triangular well in that the walls of the reservoir have a "bulge" or "bowed" portion when viewed in cross section.

The upper drum 12 is constructed of aluminum, steel or a similar material. The upper drum 12 has an open central cavity 22 which houses the at least one galvo 14. The upper drum 12 may also be configured to house a plurality of galvos, mirrors or a high density galvo block. The upper drum 12 is then rotatably secured within the assembly 10. For example, the upper drum 12 may be secured to a central support extending through the center of the upper drum 12 and positioned within the laser processing assembly 100, and about which the upper drum 12 is rotatable. The central support extends into the cavity 22 and may also support the one or more galvos 14 within the upper drum. A laser source is operably connected to the galvos 14 such that a laser beam 20 generated by the laser source is directed and/or redirected to the galvo or galvos 14 and subsequently directed to the material for selectively laser processing the material.

The upper drum 12 may be a perforated drum 12, where the exterior surface of the drum 12 has a surface topography 28 comprising a plurality of apertures 30. The surface pattern 28 covers at least a portion of the surface area of the drum 12. The plurality of apertures 30 may be spaced apart and aligned in a plurality of rows extending around the exterior surface of the drum 12. The apertures 30 are each of a size sufficient to provide an opening for the laser beam 20 which is directed from the laser source to the galvo 14 and through the corresponding aperture 30 to the material 24 positioned thereunder.

The system 10 also comprises the lower roller or drum 13 which has an external surface topography 29 that is provided with a one or more topographical features 32, such as protrusions 32 that are positioned to align with apertures 30 of the upper drum 12 when the upper drum 12 rotates. The upper drum 12 is rotatable and the lower roller or drum 13 may also rotate with the upper drum 12. The topographical surface features 32 are spaced such that during rotation the protrusions 32 are positioned near or surrounding a corresponding aperture 30 on the upper drum as the material passes between the upper drum 12 and lower roller or drum 13. The feature 32 is a protrusion such as a lip or ridge having a diameter or width that is larger than the diameter or width of the apertures 30. Thus, the protrusions 32 at least partially surround or at least partially co-extend around the perimeter of the aperture 30 but may have an inner perimeter that is spaced apart from the outer perimeter of the aperture 30. The topographical feature 32 is a raised feature relative to surface of the roller or drum 13. The feature 32 provides a directed compressive force on the material 24 as the material passes through the gap between the upper drum 12 and lower roller 13 and thus through the laser processing window.

Thus, as at least the drum 12 rotates to move the material 24 along the web and through the laser processing system 100, the laser source is operated (e.g., pulsed) to direct one laser beam 20 to the material when the laser beam 20 is for example, aligned with one aperture 30 and the material 24 there below and one protrusion 32 on the lower roller or drum 13 is positioned to surround the corresponding aperture 30. The laser beam may be otherwise aligned at an angle from the vertical with one aperture and one protrusion with the material there below. For example, the galvo may be configured to direct the laser beam through a window of size such that the laser beam can be directed substantially vertically (e.g. about 0°) or at an angle other than 0° or at an angle ranging from about 1° up to about 20° or more from the vertical in any direction.

The protrusions 32 on the lower roller or drum act as compression bumps on the lower drum 13 that work in conjunction with the apertures 32 in the upper drum 12 to provide a unique combination of compression and stretching of the material into and radially about the upper drum apertures 30. Excessive compression and or stretch results in tearing of the materials. The size of the apertures and a profile of the protrusions 32 are selected to provide and are sufficient to produce ideal reverse draft profile affect in the material. For example, the aperture may be a thin walled aperture such as an aperture with a wall of about .02" and with a diameter of .125" wherein the protrusion has a truncated conical shape with an indentation centered therein, such as a size and shape similar to that provided by an M3 button head cap screw.

The assembly 10 may be positioned along the web between one or more rollers; such as nip rollers positioned on either or both sides of the assembly 10 (down web and up web). Alternatively, the assembly 10 may be positioned within the laser processing system 100 between two nip rollers positioned on either side of the laser processing window. The nip rollers aid in movement of the material through the laser processing system 100. The rollers may also allow for separation of a carrier liner from the material prior 24 to laser processing and joining of the carrier liner with the material 24 after laser processing. The rollers also provide tension to the material 24 aiding in the compression of the material 24 near the target area 18 when processing.

Thus, the assembly 10 is configured to compress the material 24 near the target area 18 (e.g., the material at the processing site is "compressed" when the laser forms the reservoir at that processing site). Compressing the material 24 when laser processing the material allows for production of the reverse draft hole 26 while maintaining the open cell characteristics of the foam. By "reverse draft hole" is meant a well or reservoir having an inlet smaller in diameter than the reservoir or well, as illustrated in FIG. 2 and unlike the conical hole illustrated in FIG. 1 where the inlet is larger than any diameter in the conical hole. The hole or aperture 26 of this disclosure may also be referred to as a well or reservoir. In addition, by hole or aperture 26 is meant not necessarily a "through" hole or aperture, but wherein the hole or aperture may be closed at one end. The important feature of the "hole" or aperture in this disclosure is that there is an inner space whose diameter is larger than the diameter at the inlet and such that fluid is retained therein.

In one embodiment, the material 24 is perforated while continuously moving through the laser processing system 100. The material 24 is perforated at a plurality of processing sites 18. One or more galvos 14 may be used and if multiple galvos are used, they are positioned in a linear array within the drum 12 so as to perforate the material at a plurality of processing sites concurrently. Thus, multiple reverse draft holes 26 may be made concurrently and/or sequentially.

The laser beam(s) 20 is/are directed using a process referred to as "mof" (mark on the fly) which requires controlling and directing the laser beams for precision perforation along a selected pattern as the web moves. A controller 110 sends commands to the laser processing system 100 to direct and pulse the laser beam precisely as the upper drum 12 rotates so that perforations 30 are aligned with the laser beam 20 and the perforations 30 are also substantially concurrently aligned with the protrusion 32 of the lower roller or drum 13, which may also be rotating, allowing the laser beam to pass through a corresponding perforation 30 and form a reverse draft hole 26 in the compressed material 24 without the upper drum 14 passing through the path of the laser beam 20.

Further aiding compression of the material 24 as it passes through the gap between the upper drum 12 and the lower drum 13 through the laser processing window and thus through the laser processing system 100 may be an under-material air purge system 40 as described in further detail below. The air purge system 40 is positioned below the laser processing window. The lower drum 13 may house the air purge system 40 such that the lower roller or drum 13 provides compression and an air purge procedure to the material 24 passing through the laser processing window.

The under-material air purge assembly, described in co-pending U.S. Application Serial No. 15/266,852 filed on September 15, 2016 may also be provided below the material 24 and laser processing site 18 in the lower drum 13 to concurrently provide forced air directly to the material while compressing and laser processing the material. The air is selectively delivered to the processing site 18 to pressurize and/or aid in compressing the cells of the open-cell foam material during laser processing. The forced air also expels smoke and debris generated during laser processing (also referred to herein as a "plume") and provides positive pressure to the material to aid in compressing the material for laser processing the reverse draft type fluid wells or reservoirs.

The under-material air purge 40 is an assembly configured for positioning within the processing window of the laser processing system 100. The under-material air purge assembly 40 is generally positioned "under" the material being processed, or such that the material is positioned between the laser source and resulting laser beam and the air purge apparatus. For example, the polyurethane-based foam material being laser processed in a roll to roll system (e.g. continuously processed, web processing) is separated from a carrier liner, which is directed below the assembly while the material is laser processed as the material is positioned and passive over the air purge assembly.

As illustrated in FIG. 4, in general, the air purge assembly 40 comprises a tubular structure operably connected to a source of forced air 41. The assembly comprises an air chamber and the tubular structure comprises a plurality of apertures positioned such that the forced air is directed through the air chamber and out of a selected aperture or plurality of apertures. The selected aperture(s) may be those apertures positioned substantially directly in line with the axis of the laser beam and a focal point of the laser beam(s) during laser processing. Additionally, or alternatively, air may also be forced out of apertures adjacent the apertures directly below the focal point concurrently and/or sequentially. These apertures may be provided on the outside surface of the lower drum 13 and positioned within the protrusions 32 such that they can be aligned with the apertures of the upper drum 12 during compression and processing of the material.

The air flow 43 is directed out of the selected apertures in order to force the air flow directly into the foam material 24. The air is directed to an area of the material that the laser beam focal point is concurrently processing. As such, the open-cell structure of the foam material 24 allows the target area 18 of the foam material to be pressurized, even when compressed, for example, substantially concurrently with laser processing and/or directly subsequent to or directly preceding laser processing. The open cells in the foam receive the directed air flow and are pressurized as the laser is directed thereto, thus "filling" the open cells with forced (pressurized) air and minimizing the plume generated by laser processing from settling in the cells. This substantially reduces discoloration of the material due to laser processing the material.

As discussed throughout this disclosure, the assembly components may be comprised of one or more metal extrusions. The components may be comprised of aluminum or other like materials which are capable of withstanding high processing temperatures and being durable for repeated use.

## Claims

1. An assembly for producing a well or reservoir (26) in a material (24) with a laser processing system (100), the assembly comprising:
a housing (12) configured to receive a galvo therein, and wherein the first housing has exterior surface topography comprising a plurality of apertures (30);
**characterised in that** a compression mechanism (13) comprising an exterior surface topography (29) having a plurality of surface protrusions (32) thereon for compressing the material passing over at least one of the plurality of surface protrusions (32); and
wherein the housing (12) and the compression mechanism (13) are spaced apart providing a gap there between for receiving a web of the material therein.

2. The assembly of claim 1, wherein the housing (12) is a first rotatable drum and the compression mechanism (13) is a second rotatable drum.

3. The assembly of any preceding claim, wherein the exterior surface topography (29) of the compression mechanism is configured to compress the material passing through the gap and around the one or more apertures (30) in the housing (12) to compress the material within a laser processing area of the material.

4. The assembly of claim 3, wherein the laser processing area is a target area aligned with an axis of a laser beam (20) directed by the galvo through at least one aperture (30) in the housing (12) to a surface of the material compressed adjacent thereto.

5. The assembly of claim 2, wherein the second drum (13) comprises an under-material air delivery mechanism (40) therein and configured to pressurize a foam material passing through the gap by way of an aperture positioned within the protrusion.

6. The assembly of claim 5, wherein the under-material air delivery mechanism (40) is positioned within the second drum such that at least one of the plurality of apertures in the first drum (12), a protrusion on the second drum (13), and an air flow path from the air delivery mechanism (40) are all aligned with respect to the axis of the laser beam (20).

7. The assembly of any preceding claim, wherein the housing (12) comprises a perforation pattern comprising at least one row of apertures (30).

8. The assembly of any preceding claim, wherein at least one of the surface protrusions (32) of the compression mechanism (13) is configured to align with and at least partially surround a corresponding aperture perimeter to provide a compressive force to the material passing through the gap near the corresponding aperture.

9. The assembly of claim 5 or claim 6, wherein the assembly further comprises a foam material, wherein the foam material is a polyurethane based material.

10. A method of producing reservoirs (26) in an open cell foam material (24) with a laser processing system, the method comprising:
compressing the material (24) around a target area on a first surface of the material and concurrently directing a focal point of a laser beam (20) to the target area to produce the reservoirs (26) having a bulging cross section.

11. The method of claim 10, and further comprising:
passing the material through a gap between a first housing (12) and a compression mechanism (13), wherein the housing (12) is configured to receive a laser steering mechanism therein and wherein the compression mechanism (13) is configured to provide a compressive force to the material around the target area (18) passing through the gap;
aligning an aperture (30) in an external surface of the housing (12) with an external protrusion (32) on the compression mechanism (13) and further aligning the target area (18) passing through the gap therewith; and
directing the focal point of the laser beam (20) from the housing and through the aperture and to the target area (18) compressed by the compression mechanism for producing the reservoir (26).

12. The method of claim 11, wherein the housing is a rotatable housing have a plurality of protrusions spaced apart on the external surface thereof and the compression mechanism (13) is movable and comprises a plurality of external protrusions (32) and concurrently rotating the housing and moving the compression mechanism for continuously aligning an aperture (30) in the external surface of the housing with a protrusion on the compression mechanism for producing a plurality of reservoirs (26) on a moving web of material.

13. The method of claim 11, further comprising aligning an axis of the laser beam (20) with the aperture and target area (18).

14. The method of any one of claims 10 to 13, further comprising providing a source of forced air below a travel path of the material through the gap for pressurizing the material at or near the target area (18) for preventing a plume generated from laser processing from settling on the material.

15. The method of claim 14, wherein laser processing the material, compressing the material and pressurizing the material occur at the target area (18) and approximately concurrently.

## Patentansprüche

1. Anordnung zum Herstellen einer Vertiefung oder eines Reservoirs (26) in einem Material (24) mit einem Laserbearbeitungssystem (100), wobei die Anordnung umfasst:
ein Gehäuse (12), das dafür ausgelegt ist, ein Galvanometer darin aufzunehmen, und wobei das erste Gehäuse eine Außenflächentopographie aufweist, die eine Vielzahl von Öffnungen (30) umfasst;
**dadurch gekennzeichnet, dass** ein Kompressionsmechanismus (13) eine Außenflächentopographie (29) mit einer Vielzahl von Oberflächenvorsprüngen (32) darauf umfasst, um das Material, das über mindestens einen der Vielzahl von Oberflächenvorsprüngen (32) läuft, zu komprimieren; und
wobei das Gehäuse (12) und der Kompressionsmechanismus (13) voneinander beabstandet sind, wodurch dazwischen ein Spalt zur Aufnahme einer Bahn des Materials darin bereitgestellt wird.

2. Anordnung nach Anspruch 1, wobei das Gehäuse (12) eine erste drehbare Trommel und der Kompressionsmechanismus (13) eine zweite drehbare Trommel ist.

3. Anordnung nach einem der vorhergehenden Ansprüche, wobei die Außenflächentopographie (29) des Kompressionsmechanismus dafür ausgelegt ist, das Material, das durch den Spalt und um die eine oder die mehreren Öffnungen (30) im Gehäuse (12) herum läuft, zu komprimieren, damit das Material innerhalb eines Laserbearbeitungsbereichs des Materials komprimiert wird.

4. Anordnung nach Anspruch 3, wobei der Laserbearbeitungsbereich ein Zielbereich ist, der mit einer Achse eines Laserstrahls (20) ausgerichtet ist, der durch das Galvanometer durch mindestens eine Öffnung (30) im Gehäuse (12) auf eine dazu benachbarte Oberfläche des komprimierten Materials gerichtet ist.

5. Anordnung nach Anspruch 2, wobei die zweite Trommel (13) innen einen Mechanismus zur Luftzufuhr unter dem Material (40) umfasst und dafür ausgelegt ist, ein Schaumstoffmaterial, das durch den Spalt hindurchgeht, durch eine Öffnung, die innerhalb des Vorsprungs angeordnet ist, mit Druck zu beaufschlagen.

6. Anordnung nach Anspruch 5, wobei der Mechanismus zur Luftzufuhr unter dem Material (40) innerhalb der zweiten Trommel derart angeordnet ist, dass mindestens eine der Vielzahl von Öffnungen in der ersten Trommel (12), ein Vorsprung an der zweiten Trommel (13) und ein Luftstromweg vom Luftzufuhrmechanismus (40) alle in Bezug auf die Achse des Laserstrahls (20) ausgerichtet sind.

7. Anordnung nach einem der vorhergehenden Ansprüche, wobei das Gehäuse (12) ein Perforationsmuster aufweist, das mindestens eine Zeile von Öffnungen (30) umfasst.

8. Anordnung nach einem der vorhergehenden Ansprüche, wobei mindestens einer der Oberflächenvorsprünge (32) des Kompressionsmechanismus (13) dafür ausgelegt ist, mit einem entsprechenden Öffnungsumfang ausgerichtet zu sein und diesen mindestens teilweise zu umgeben, um eine Kompressionskraft auf das Material, das durch den Spalt in der Nähe der entsprechenden Öffnung hindurchgeht, bereitzustellen.

9. Anordnung nach Anspruch 5 oder 6, wobei die Anordnung ferner ein Schaumstoffmaterial umfasst, wobei das Schaumstoffmaterial ein Material auf Polyurethanbasis ist.

10. Verfahren zum Herstellen von Reservoiren (26) in einem offenzelligen Schaumstoffmaterial (24) mit einem Laserbearbeitungssystem, wobei das Verfahren umfasst: Komprimieren des Materials (24) um einen Zielbereich auf einer ersten Oberfläche des Materials und gleichzeitiges Richten eines Brennpunkts eines Laserstrahls (20) auf den Zielbereich zum Erzeugen der Reservoire (26) mit einem gewölbten Querschnitt.

11. Verfahren nach Anspruch 10, ferner umfassend:
Hindurchführen des Materials durch einen Spalt zwischen einem ersten Gehäuse (12) und einem Kompressionsmechanismus (13), wobei das Gehäuse (12) dafür ausgelegt ist, einen Laserführungsmechanismus darin aufzunehmen, und wobei der Kompressionsmechanismus (13) dafür ausgelegt ist, eine Kompressionskraft auf das Material um den durch den Spalt hindurchgehenden Zielbereich (18) bereitzustellen;
Ausrichten einer Öffnung (30) in einer Außenfläche des Gehäuses (12) mit einem äußeren Vorsprung (32) am Kompressionsmechanismus (13) und weiteres Ausrichten des durch den Spalt hindurchgehenden Zielbereichs (18) damit; und
Richten des Brennpunkts des Laserstrahls (20) aus dem Gehäuse und durch die Öffnung und auf den Zielbereich (18), der durch den Kompressionsmechanismus komprimiert wird, zum Erzeugen des Reservoirs (26).

12. Verfahren nach Anspruch 11, wobei das Gehäuse ein drehbares Gehäuse ist, das eine Vielzahl von Vorsprüngen aufweist, die auf seiner Außenfläche voneinander beabstandet sind, und der Kompressionsmechanismus (13) beweglich ist und eine Vielzahl von äußeren Vorsprüngen (32) umfasst und gleichzeitig das Gehäuse dreht und den Kompressionsmechanismus bewegt, um eine Öffnung (30) in der Außenfläche des Gehäuses kontinuierlich mit einem Vorsprung am Kompressionsmechanismus auszurichten, um eine Vielzahl von Reservoiren (26) auf einer sich bewegenden Bahn von Material zu erzeugen.

13. Verfahren nach Anspruch 11, ferner umfassend das Ausrichten einer Achse des Laserstrahls (20) mit der Öffnung und dem Zielbereich (18).

14. Verfahren nach einem der Ansprüche 10 bis 13, ferner umfassend das Bereitstellen einer Druckluftquelle unterhalb eines Bewegungswegs des Materials durch den Spalt zur Druckbeaufschlagung des Materials an oder nahe dem Zielbereich (18), um zu verhindern, dass sich eine bei der Laserbearbeitung erzeugte Rauchfahne auf dem Material absetzt.

15. Verfahren nach Anspruch 14, wobei die Laserbearbeitung des Materials, das Komprimieren des Materials und die Druckbeaufschlagung des Materials am Zielbereich (18) und ungefähr gleichzeitig erfolgen.

## Revendications

1. Ensemble pour la production d'un puits ou d'un réservoir (26) dans un matériau (24) avec un système de traitement laser (100), l'ensemble comprenant :
un boîtier (12) configuré pour recevoir un galvanomètre dans celui-ci, et le premier boîtier ayant une topographie de surface extérieure comprenant une pluralité d'ouvertures (30) ;
**caractérisé en ce qu'**un mécanisme de compression (13) comprend une topographie de surface extérieure (29) ayant une pluralité de saillies de surface (32) sur celle-ci pour comprimer le matériau passant sur au moins une de la pluralité de saillies de surface (32) ; et
le boîtier (12) et le mécanisme de compression (13) étant espacés l'un de l'autre, ce qui laisse un espace entre eux pour recevoir une bande de matériau.

2. Ensemble selon la revendication 1, le boîtier (12) étant un premier tambour rotatif et le mécanisme de compression (13) étant un second tambour rotatif.

3. Ensemble selon n'importe quelle revendication précédente, la topographie de surface extérieure (29) du mécanisme de compression étant configurée pour comprimer le matériau passant à travers l'espace et autour de la ou des ouvertures (30) dans le boîtier (12) pour comprimer le matériau dans une zone de traitement laser du matériau.

4. Ensemble selon la revendication 3, la zone de traitement laser étant une zone cible alignée avec un axe d'un faisceau laser (20) dirigé par le galvanomètre à travers au moins une ouverture (30) dans le boîtier (12) vers une surface du matériau comprimé adjacente à celle-ci.

5. Ensemble selon la revendication 2, le second tambour (13) comprenant un mécanisme de distribution d'air sous le matériau (40) dans celui-ci et configuré pour pressuriser un matériau de mousse passant à travers l'espace par une ouverture positionnée dans la saillie.

6. Ensemble selon la revendication 5, le mécanisme de distribution d'air sous le matériau (40) étant positionné à l'intérieur du second tambour de telle sorte qu'au moins une de la pluralité d'ouvertures dans le premier tambour (12), une saillie sur le second tambour (13), et un chemin d'écoulement d'air à partir du mécanisme de distribution d'air (40) sont tous alignés par rapport à l'axe du faisceau laser (20).

7. Ensemble selon n'importe quelle revendication précédente, le boîtier (12) comprenant un motif de perforation comprenant au moins une rangée d'ouvertures (30).

8. Ensemble selon n'importe quelle revendication précédente, au moins une des saillies de surface (32) du mécanisme de compression (13) étant configurée pour s'aligner avec et entourer au moins partiellement un périmètre d'ouverture correspondant pour fournir une force de compression au matériau passant à travers l'espace près de l'ouverture correspondante.

9. Ensemble selon la revendication 5 ou la revendication 6, l'ensemble comprenant en outre un matériau de mousse, le matériau de mousse étant un matériau à base de polyuréthane.

10. Procédé de production de réservoirs (26) dans un matériau de mousse à cellules ouvertes (24) avec un système de traitement laser, le procédé comprenant :
la compression du matériau (24) autour d'une zone cible sur une première surface du matériau et l'orientation simultanément d'un point focal d'un faisceau laser (20) vers la zone cible pour produire les réservoirs (26) ayant une section transversale bombée.

11. Procédé selon la revendication 10, et comprenant en outre :
le passage du matériau à travers un espace entre un premier boîtier (12) et un mécanisme de compression (13), le boîtier (12) étant configuré pour recevoir un mécanisme de direction laser dans celui-ci et le mécanisme de compression (13) étant configuré pour fournir une force de compression au matériau autour de la zone cible (18) passant à travers l'espace ;
l'alignement d'une ouverture (30) dans une surface externe du boîtier (12) avec une saillie externe (32) sur le mécanisme de compression (13) et l'alignement supplémentaire de la zone cible (18) passant à travers l'espace avec celle-ci ; et
l'orientation du point focal du faisceau laser (20) à partir du boîtier et à travers l'ouverture vers la zone cible (18) comprimée par le mécanisme de compression pour produire le réservoir (26).

12. Procédé selon la revendication 11, le boîtier étant un boîtier rotatif ayant une pluralité de saillies espacées sur sa surface externe et le mécanisme de compression (13) étant mobile et comprenant une pluralité de saillies externes (32), et tournant le boîtier et déplaçant simultanément le mécanisme de compression pour aligner en continu une ouverture (30) dans la surface externe du boîtier avec une saillie sur le mécanisme de compression pour produire une pluralité de réservoirs (26) sur une bande de matériau en mouvement.

13. Procédé selon la revendication 11, comprenant en outre l'alignement d'un axe du faisceau laser (20) avec l'ouverture et la zone cible (18).

14. Procédé selon l'une quelconque des revendications 10 à 13, comprenant en outre la fourniture d'une source d'air forcé sous un chemin de déplacement du matériau à travers l'espace pour pressuriser le matériau au niveau ou à proximité de la zone cible (18) pour empêcher un panache généré par le traitement laser de se déposer sur le matériau.

15. Procédé selon la revendication 14, le traitement laser du matériau, la compression du matériau et la pressurisation du matériau se produisant au niveau de la zone cible (18) et approximativement simultanément.
